(19) Europäisches Patentamt
European Patent Office
Office européen des brevets

(11) **EP 1 207 929 B1**

(12) **EUROPEAN PATENT SPECIFICATION**

(45) Date of publication and mention
of the grant of the patent:
**30.05.2012 Bulletin 2012/22**

(51) Int Cl.:
*A61M 16/16* (2006.01)

(21) Application number: **00955190.4**

(22) Date of filing: **09.08.2000**

(86) International application number:
**PCT/NZ2000/000156**

(87) International publication number:
**WO 2001/013981 (01.03.2001 Gazette 2001/09)**

(54) **Humidity controller**

Vorrichtung zur Feuchtigkeitskontrolle

Controleur d'humidité

(84) Designated Contracting States:
**AT BE CH CY DE DK ES FI FR GB GR IE IT LI LU MC NL PT SE**

(30) Priority: **23.08.1999 NZ 33738299**

(43) Date of publication of application:
**29.05.2002 Bulletin 2002/22**

(73) Proprietor: **Fisher & Paykel Healthcare Limited East Tamaki,
Auckland (NZ)**

(72) Inventors:
• **THUDOR, Mohammad**
  **Auckland (NZ)**
• **WIXEY, David**
  **Auckland (NZ)**
• **HUNT, Peter, John**
  **Auckland (NZ)**

(74) Representative: **Hoarton, Lloyd Douglas Charles et al**
**Forresters**
**Skygarden**
**Erika-Mann-Strasse 11**
**80636 München (DE)**

(56) References cited:
**EP-A- 0 845 277      EP-A- 0 885 623
EP-A- 1 127 583      EP-A2- 0 885 623
WO-A-01/41854      WO-A-97/10019
AU-A- 1 486 395      GB-A- 1 294 808
GB-A- 2 192 136      GB-A- 2 338 420
GB-A- 2 338 420      JP-A- 5 317 428
US-A- 4 621 632      US-A- 4 682 010
US-A- 5 031 612      US-A- 5 558 084
US-A- 5 626 129**

Description

TECHNICAL FIELD

This invention relates to breathing assistance apparatus, particularly but not solely, for supplying optimal humidity temperature of gases to a patient to assist the patient's breathing.

BACKGROUND ART

A number of methods are known in the art for assisting a patient's breathing. Continuous Positive Airway pressure or CPAP involves the administration of air under pressure to a patient, usually by a nasal mask. It is used in the treatment of snoring and Obstructive Sleep Apnea (OSA), a condition characterised by repetitive collapse of the upper airway during inspiration. Positive pressure splints the upper airway open, preventing its collapse. Treatment of OSA with nasal CPAP has proven to be both effective and safe, but CPAP is difficult to use and the majority of patients experience significant side effects, particularly in the early stages of treatment.

Upper airway symptoms adversely affect treatment with CPAP. Mucosal drying is uncomfortable and may awaken patients during the night. Rebound nasal congestion commonly occurs during the following day, simulating a viral infection. If untreated, upper airway symptoms adversely affect rates of CPAP use.

Increases in nasal resistance may affect the level of CPAP treatment delivered to the pharynx, and reduce the effectiveness of treatment. An individual pressure is determined for each patient using CPAP and this pressure is set at the mask. Changes in nasal resistance affect pressure delivered to the pharynx and if the changes are of sufficient magnitude there may be recurrence of snoring or airway collapse.

Such symptoms can also occur in a hospital environment where a patient is on a respirator. Typically in such situations the patient is intubated. Therefore the throat tissue may become irritated and inflamed causing both distress to the patient and possible further respiratory problems.

A number of methods may be employed to treat such upper airway symptoms, including pharmacologic agents to reduce nasal disease, or heating the bedroom. One most commonly employed method is humidification of the inspired air using an in line humidifier. Two types of humidifier are currently used. Cold passover humidifiers rely on humidifying the air through exposure to a large surface area of water. While they are cheap, the humidity output is low at high flows, typically 2 to 4 mg\L absolute humidity at flows above 25L/min. The output is insufficient to prevent mucosal drying. Heated water bath humidifiers are more efficient, and produce high levels of humidity even at high flow rates. They are effective at preventing upper airway mucosal drying, prevent increases in nasal resistance, and are the most reliable means of treating upper airway symptoms.

Any of these active systems will have, to some degree or other, condensation (or rain out) in the tubing connecting the humidifier to the patient. The degree of condensation is strongly dependent on the ambient temperature, being much greater for greater differences between the ambient temperature and the gas temperature. The formation of large quantities of water in the breathing tubing causes considerable inconvenience to the patient, may accelerate cooling of the gas, may eventually occlude the tubing, or may be expelled into the patient. Also, the patient may experience discomfort, when breathing gases are delivered at temperatures widely divergent from that of the ambient temperature. Excessive condensation also results in inefficient usage of the water in the humidifying chamber.

In a hospital environment, where the ambient temperature of the atmosphere within the hospital environment is controlled by air conditioning for example, the required temperature for the humidified gases supplied by the apparatus may be controlled within set temperature parameters that are sufficiently close to the ambient temperature to prevent condensation within the conduit. However it is still necessary to have good control over the temperature and humidity of gases as they are actually supplied to the patient.

In the home care environment in which a user requires to use humidifying apparatus at home, the range of ambient and gas temperatures may well exceed that of the hospital environment. In the home care environment, the user will usually wear a face mask which is connected to end of the conduit and such a humidifier may be used in the home environment for the treatment of breathing and sleep apnea disorders and/or in conjunction with ventilators or CPAP devices. In addition, non active humidifiers are commonly employed utilising the known pass over humidification technique.

EP0885623 A2 discloses a flow sensor for use in a respiratory humidification system. The flow sensor comprises essentially two temperature sensors. The measurement of flow is described as useful in a number of control strategies for the humidification system.

In US Pat. No. 5640951 issued to Fisher and Paykel a heated conduit for a humidified breathing assistance apparatus is disclosed which includes a temperature probe at the end of a heated conduit. By heating the conduit the problems relating to condensation in the conduit may be overcome. However in order to implement closed loop control over the temperature of the supplied gases (and therefore the power input to the conduit heater element), it is necessary to measure the temperature as close to the point at which it is supplied as possible. The temperature probe and its associated wiring included for this purpose make the attachment to the face mask or intubated patient bulky and therefore more uncomfortable for the patient. Therefore it would be advantageous if a heated conduit for a humidified breathing assistance apparatus could be implemented without the need for a temperature probe at the end of the conduit. It would also be advantageous to have some indication, when the conduit heater is energised, that it is operating correctly.

Embodiments of the present invention seek to provide a breathing assistance apparatus which goes some way to overcoming the abovementioned disadvantages or which at least provides the public or industry with a useful choice.

Accordingly in a first aspect, the invention consists of a breathing assistance apparatus adapted to deliver humidified gases at a desired level of humidity or at a desired temperature according to any one of claims 1 to 9 hereinafter.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

## BRIEF DESCRIPTION OF THE DRAWINGS

One preferred form of the present invention will now be described with reference to the accompanying drawings in which;

Figure 1 is a illustration of a respiratory humidifier system,
Figure 2 is a illustration of the humidifier base of the respiratory humidifier system of Figure 1,
Figure 3 is a block diagram of the control system which controls the humidifier in the preferred embodiment of the present invention,
Figure 4 is a flow diagram of the algorithm used to control the heater wire within the respiratory conduit,
Figure 5 is an example of how the heater plate temperature varies over time, when the pressure is controlled constant,
Figure 6 is a graph of heater plate power against flow rate, and
Figure 7 is a graph of conduit heater element power and flow rate.

## DETAILED DESCRIPTION OF THE INVENTION

Whether used in a hospital environment or in a home care environment, the present invention will generally have associated two main pieces of apparatus. Firstly an active humidifier which controls the temperature of a heater plate heating a body of water to achieve a desired temperature and humidity of the gases being humidified. Secondly a transport conduit from the humidifier to the patient is also required, which is preferably heated to reduce condensation, or "rain out".

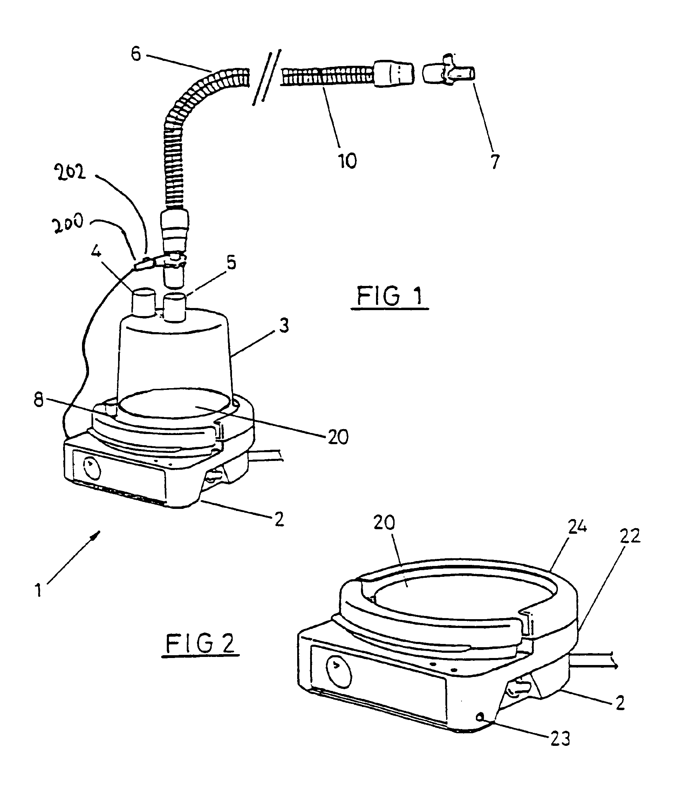

Referring to Figure 1 a humidifying apparatus as might be used in a hospital generally referenced 1 is shown. The apparatus comprises a body 2 containing heating means comprising a heating plate 20 having an electric heating element therein or in thermal contact therewith and control means for example electronic circuitry which may include a microprocessor for controlling the supply of energy to the heating element. The body 2 is removably engageable with a humidifying chamber 3 which contains water for humidifying gases. Referring to Figures 2 which show the humidifier apparatus in more detail, the humidifying chamber 3 has edges which engage with collar 24 on the humidifier apparatus. The gases to be humidified may be a mixture of air, oxygen and anaesthetic for example which are supplied to the chamber through a gases inlet 4. This might be connected to a ventilator, or in the case of CPAP therapy a CPAP blower. A gases outlet 5 is also provided and the gases outlet 5 is connected to the conduit 6 (Figure 1) which conveys humidified gases to a remote destination such as an intubated patient at the end 7 of the conduit. Alternatively, the end 7 of the conduit may have a gas mask attached thereto, which mask is used to cover a nose and/or mouth of a user so as to supply humidified gases to the user for breathing, as in the delivery of CPAP therapy. The humidifier heater plate 20 has a temperature transducer 8 which is in electrical connection with the electronic control circuitry in body 2 of the apparatus so that the control means monitors the temperature of the heating plate.

A heating element 10 is provided within the conduit 6 to help prevent condensation of the humidified gases within the conduit. Such condensation is due to the temperature of the walls of the conduit being close to the ambient temperature, (being the temperature of the surrounding atmosphere) which is usually lower than the temperature of the humidified gases within the conduit. The heater element is effectively replaces the energy lost from the gases through conduction and convection during transit through the conduit. Thus the conduit heater element ensures the gases delivered are at an optimal temperature and humidity.

The present invention provides a means of controlling at least the heater plate and preferably also the conduit heater element without the need for any sensors, either in the humidifier chamber or positioned in the conduit. This is achieved by estimating the rate of flow of gases through the humidifier using parameters already available to the controller.

For a given humidifier an appropriate level of power can then be determined to apply to the heater plate to achieve the desired temperature of gases delivered to the patient. Additionally this may be used to provide a more appropriate level of energisation at this conduit heater element. This not only saves the cost of the extra sensors but also allows the apparatus connected to the end of the conduit to be simpler and lighter.

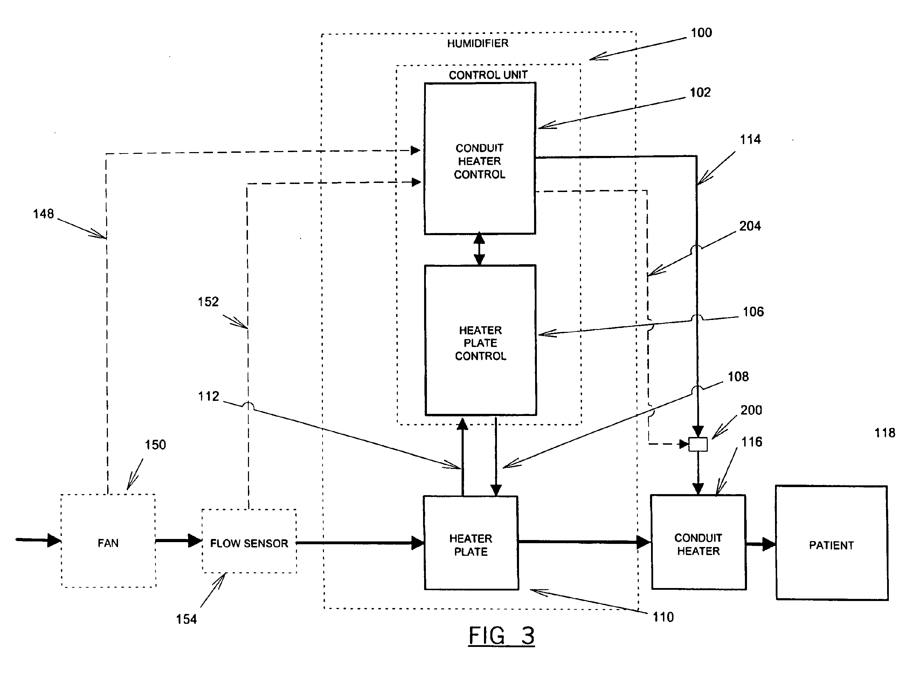

In the preferred embodiment of the present invention the controller 100, shown in Figure 3, uses a range of inputs to control both the power 108 supplied to the heater plate 110 as well as the power 114 supplied to the conduit heating element 116 (if present). In certain applications it may also be used to provide control instructions to auxiliary apparatus such as a blower fan. Using an internal algorithm 106 the controller 100 estimates the power 108 to supply to the humidifier heater plate 110 to achieve a given humidity and or temperature of gases at the top of the humidifier chamber alternatively (or estimates the temperature to achieve a given power). It then uses a second algorithm 102 to estimate the required power 114 to supply to the conduit heater element 116 and the humidifier heater plate 110 to achieve optimal temperature and/or humidity of the gases delivered to the patient 118.

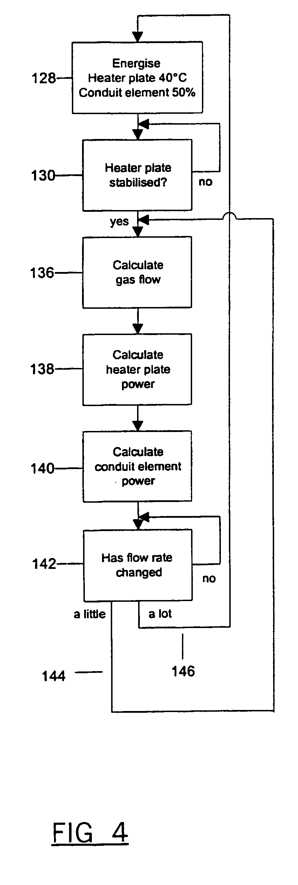

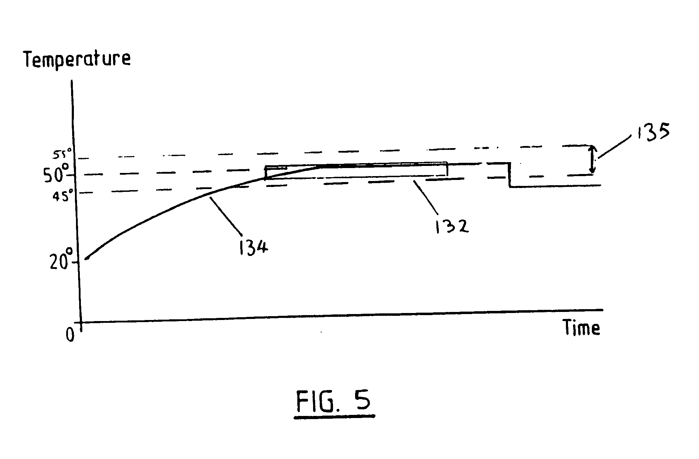

Referring to Figure 4, when the humidifier starts up the controller executes a supervisory algorithm, which controls the heater plate and if present the conduit heater element. Initially 128 the heater plate is controlled to a temperature of 40°C and the conduit heater element may be energised with a duty cycle of for example 50%. The heater plate temperature (or alternatively the power supplied to the heater plate) is then monitored 130 until it settles to a stabilised level. Effectively a window 132 is superimposed over the heater plate temperature profile 134 of which an example is shown in Figure 5. When the profile 134 (over the entire period of the window 132) fits within the bounds of the window 132, it is effectively considered to have stabilised. Once this has occurred the controller enters a calculation stage.

Firstly, it calculates the flow rate of the gases 136 using any one of a number of methods which will be described later.

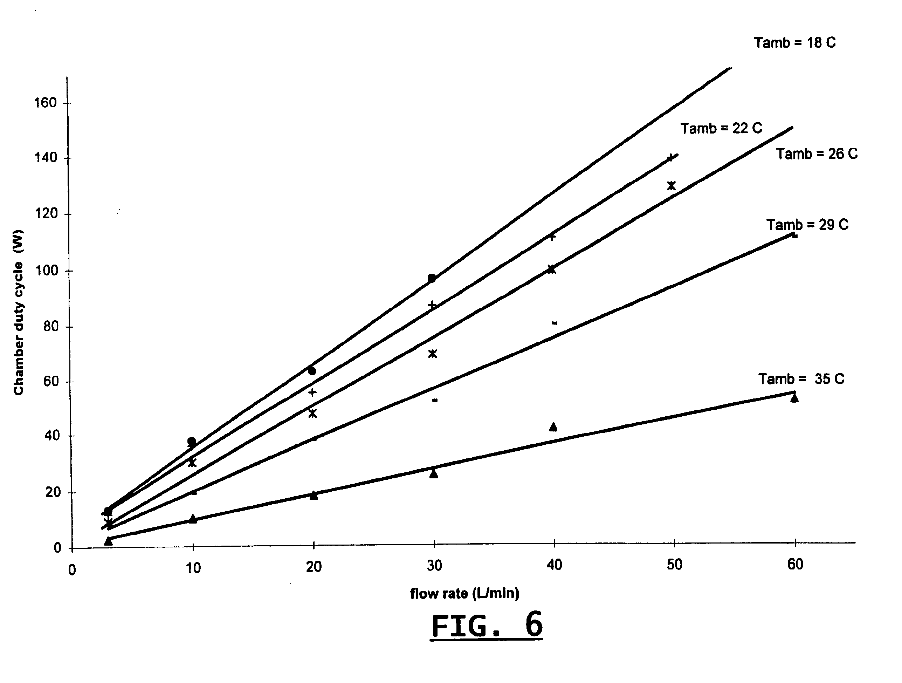

Secondly knowing the rate of flow of the gases the algorithm then calculates the required heater plate power 138 (alternatively heater plate temperature) to achieve a desired temperature/humidity of gases (alternatively heater plate power). A relationship has been empirically determined using a humidifier and a heated conduit such as that as described in US5640951. The actual relationship for any other arrangement would either have to be empirically determined by experimentation or theoretically calculated. For a desired temperature of gases exiting the humidifier of for example 37°C the relationship between the power supplied to the heater plate ($P_{HP}$), the rate of flow of gases ($F_{gas}$) and the ambient temperature ($T_{amb}$) is graphed in Figure 6. From this an approximate general algebraic equation has been extrapolated which the controller can use to determine an approximate level of power to apply to the heater plate:

$$P_{HP} = (-0.1239 \times T_{amb} + 5.383) \times F_{gas} + (-0.3112 \times T_{amb} + 10.738)$$

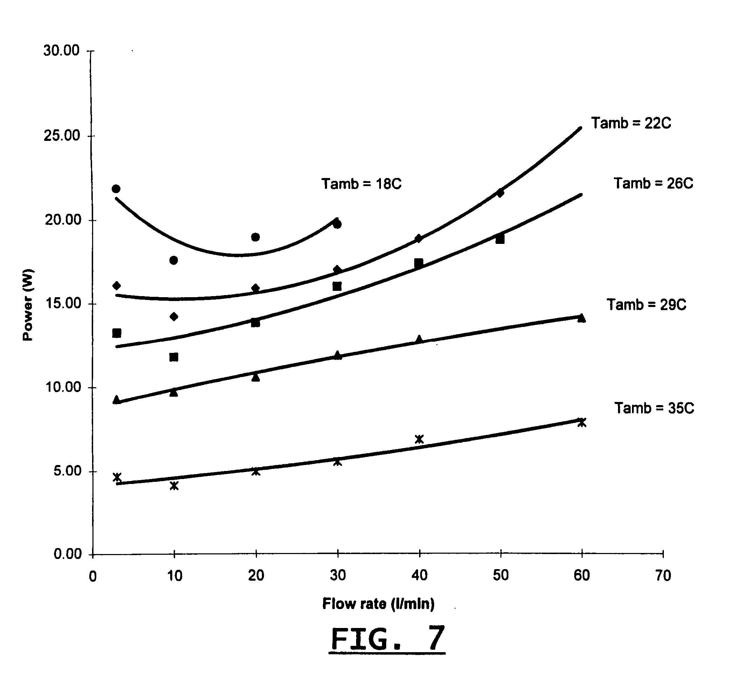

Thirdly the algorithm calculates the required power input to the conduit heater wire 140 to deliver a desired temperature of the gases to the patient. With gases flowing at a known rate of flow it is possible to calculate the resultant temperature of the gases once they have flowed through a conduit of known characteristics surrounded by the atmosphere at a known or assumed ambient temperature. Thermal characteristics of the conduit will either be known or can be calculated by experimentation. This relationship is based off empirical data using a humidifier and a heated conduit such as that as described in US5640951. The actual relationship for any other arrangement would either have to be empirically determined by experimentation or theoretically calculated. With a conduit entry gas temperature of 37°C and a temperature of gases delivered to the patient of 40°C, the relationship between the flow rate of the gases ($F_{gas}$), the power input to the conduit heater element ($P_c$), the ambient temperature ($T_{amb}$) is graphed in Figure 7. This is extrapolated to a general algebraic expression:

$$P_c = (-0.0005 * T_{amb} + 0.0169) F_{gas}^2 - [10^{-5} * T_{amb}^3 - 0.0042 * T_{amb}^2 + 0.2189 * T_{amb} - 3.0075] F_{gas} - 1.0169 * T_{amb} + 38.956$$

Practically this relationship can be simplified whereby $P_c$ is dependent only on $T_{amb}$. This is an acceptable approximation for the conduit heater element, as it is not as crucial as the heater plate.

Once the heater plate and conduit heater element have been appropriately energised, the controller continues to monitor 142 the system for any changes in the variables. The main reason for this is to avoid thermal overshoot ie where the flow drops suddenly, the temperature of gases can become dangerously high.

In order to monitor effectively, two methods are used. Firstly the flow rate is monitored and secondly the change in flow rate (with respect to time) is also monitored. The first 144 is to allow the system to respond to any changes in the system. The second 146 is a fast response system in order to avoid thermal overshoot. Effectively where either $P_{HP}$

or $T_{HP}$ is controlled constant, monitoring the other variable gives an indication of any change in flow, or any other variable which requires a recalculation.

In order to monitor the flow a variable x (defined as $P_{HP}/T_{HP}$), which is closely related to the flow rate, is constantly calculated and monitored. If it goes up there is a 30 minute delay before the controller initiates a recalculation, to avoid spurious readings and unnecessary calculations. If it goes down there is a 30 second delay before the controller recalculates, to avoid any possibility of the delivered gases being, even transiently, too hot.

Where large step changes occur the controller needs to react quickly. In such cases it will reset to initial conditions to wait until the system stabilises again, as any calculations in the interim would be pointless. To achieve this dx/dt is calculated and monitored. While a negative value is more dangerous, any deviation over a certain value will reset the controller.

In an alternative embodiment of the present invention the expected heater plate temperature is calculated using

$$T_{HP} = -7.3319*Ln(F_{gas}) + 63.655$$

and if the actual heater plate temperature deviates by more than 5°C then the program recalculates the required powers.

Thus in summary controller carries out the following steps:

1) Estimates the rate of flow of gases keeping all variables constant 136.
2) Estimate the required heater plate power/temperature to achieve a specified temperature/humidity of gases in the humidification chamber 138.
3) Calculate the power input to the heater wire to achieve a desired output temperature 140.

It will be appreciated that a greater level of power will be supplied to the conduit heater element if:

i) the rate of flow of the gases reduces,
ii) the ambient temperature decreases,
iii) the differential between ambient and gases temperature increases.

It will also be appreciated that the heater plate temperature could be controlled to a set value (using closed loop control) as opposed to power. In this case the power supplied would be monitored as a measure of system stability. Furthermore where relationships are expressed algebraically they could equally be stored in look-up tables.

## Preferred embodiment of flow estimation

Generally when used in a hospital setting a humidifier such as that described in the present invention will be used in conjunction with a respirator to supply humidified gases to an intubated patient, or possibly using a respiratory mask. As such the humidifier will operate effectively independently of the respirator and therefore must make all of its control decisions based on only the sensors contained therein. In the preferred embodiment of the present invention the flow rate of the gases passing through the humidification chamber can first be estimated by comparing the power input required 108 for the humidifier heater plate to the measured temperature 112 of the heater plate. In effect the higher the rate of flow of gases the larger the amount of power required by the heater plate in order to achieve a given heater plate temperature. Thus for a given system the relationship between power to heater plate and flow rate for a given heater plate temperature can either be determined empirically or theoretically calculated. Again using a humidifier and a heated conduit such as that as described in US5640951 1 the following empirically determined relationship applies:

$$F_{gas} = \frac{-(0.831 - 0.0049*T_{amb}) + \sqrt{abs\left[(0.831 - 0.0049*T_{amb})^2 - \left(4*(0.00004*T_{amb} - 0.0057)*\left((14.348 - 0.25*T_{amb}) - P_{HP}\right)\right)\right]}}{2*(0.0004*T_{amb} - 0.0057)}$$

where $P_{HP}$ is the power applied to the heater plate to achieve a given heater plate temperature in steady state of 50°C, $T_{amb}$ is the ambient temperature and $F_{gas}$ is the gas flow rate.

It will be appreciated this method is more appropriate in the hospital care environment where the ambient temperature can be assured with a high degree of confidence.

**Alternative means of Flow Estimation**

In the homecare environment the present invention will often be employed in conjunction with a continuous positive airway pressure (CPAP) device or such other breathing apparatus which will include a fan such as that described in US Patent No. 6050260. It will be appreciated that in such applications it may be possible to connect the controllers of the various devices together in an arrangement such that data may be readily exchanged. In such cases the rate of flow of the gases may be estimated directly from information available either from the fan or, where provided, a flow sensor.

In this alternative means of flow estimation the flow is estimated based on the loading of the fan. Generally the fan will be controlled to run at a specified speed and therefore deliver a constant pressure output. The flow rate of the gases will depend on the restrictions in the flow path. In turn in order to maintain the specified speed a certain power input will be required for the fan. Therefore an algebraic relationship between the actual gas flow rate and the power input to the fan can be developed for a fan of known characteristics. This relationship may either be determined empirically by experimentation or theoretically calculated using specified motor characteristics.

A number of methods are known in the art for determining the loading on a motor from the supply it draws. The simplest such method would be to firstly meter the current drawn 148 from the fan 150, as indicated in Figure 3. The current 148 is the input to the conduit heater element controller 102 where either an algebraic relationship or a look up table is used to determine the flow rate of the gases.

For example in US5740795, a method is disclosed using both motor voltage and current to estimate the flow rate. While this represents one method, as mentioned above, it will be appreciated that other methods, such as based on just current, will be equally applicable.

**Second alternative means of Flow Estimation**

As mentioned in the second embodiment that in certain cases a flow sensor may already be provided in the gas flow path. This being the case, the gas flow rate 152 can be extracted directly from the flow sensor 154 and used as an input to the humidifier controller 100, as indicated in Figure 3. This is then used directly in the conduit heater element controller 102 to determine the power to apply to the heater plate 110 and conduit heater element 116 according to the algorithm shown in Figure 4 and described earlier. However, this is outside the scope of the present invention.

**Heater Wire Adaptor**

In order to connect the conduit heater element to the power supply in the humidifier, an adaptor cable is required. In the preferred embodiment of the present invention, the adaptor 200 includes an indicator 202 to indicate whether the conduit heater element is operating correctly, when the adaptor is plugged in, as shown in Figure 1.

The humidifier controller continually detects for the conduit heater element and determines whether it is operating correctly. It does this by energising the conduit heater element intermittently, and if the expected current results it energises 204 the indicator (eg an LED).

The present invention as described in the foregoing provides a novel method and apparatus for controlling the heater plate temperature in a humidifier for supplying humidified gases to a patient under respiratory therapy. This has the advantage of removing external sensors making the system simpler, cheaper and lighter. Similarly it may also allow for effective control over energisation of the conduit heater element, ensuring the system as a whole operates correctly as well as being as efficient as possible.

**Claims**

1. A breathing assistance apparatus adapted to deliver humidified gases at a desired level of humidity or at a desired temperature comprising:

    humidification means (1) comprising a humidification chamber (3) adapted to receive a volume of water and water heating means (20) to heat said water to produce water vapour within said chamber in use, said gases passing through said water vapour in said chamber thereby being humidified, said humidification means having an electrical input power and capable of humidifying said gases up to a level of humidity, said level of humidity depending on said input power to said humidification means, and

    chamber sensing means (8) providing an indication of the temperature of said water heating means (20) and providing an indication of the electrical power drawn by said water heating means **characterised by**,
    control means (100) including stored instructions to:

(a) determine a parameter relating to the flow rate of said gases through said apparatus, said instruction (a) comprising;

i) energising said water heating means to heat said water towards a first condition,

ii) continuously monitoring a variable indicative of a property of said water heating means, until said variable indicates that said water has substantially reached said first condition, said variable being indicative of said indication of the temperature of said water heating means or said indication of the power drawn by said water heating means, and

iii) determining said parameter based on at least said variable,

(b) determine based on at least said parameter the required electrical power input to said humidification means to deliver said gases at a level of humidity or at a temperature substantially similar to said desired level of humidity or said desired temperature; and

(c) supply as said input power to said humidification means a level of power substantially similar to said determined power input to said humidification means.

2. A breathing assistance apparatus according to claim 1, further comprising transportation pathway means (6) for conveying said humidified gases from said humidification means to a patient.

3. A breathing assistance apparatus as claimed in claim 2 further comprising:

pathway heating means (10) having an electrical input power, and being associated with said transportation pathway means (6) wherein gases flowing through said transportation pathway means are heated, either directly or indirectly, by said pathway heating means, the level of heating depending on said input power to said pathway heating means; and

an ambient temperature sensor providing an indication of the exterior temperature;

wherein said instruction (b) further comprises an instruction to determine, based on at least said indication of the exterior temperature, the required power input to said pathway heating means to deliver said gases to said patient at a level of humidity or at a temperature substantially similar to said desired level of humidity or said desired temperature;

and wherein said instruction (c) further comprises an instruction to supply as said input power to said pathway heating means a level of power substantially similar to said determined power input to said pathway heating means.

4. A breathing assistance apparatus as claimed in claim 3 wherein the determination of said power to said humidification means in instruction (b) is based on said indication of the exterior temperature.

5. A breathing assistance apparatus as claimed in claim 3 wherein said control means stores a further instruction to:

(d) continuously monitor said parameter or said variable, and when a change in said parameter or said variable is greater than a first threshold, revert to said instruction (b), and when a change in said parameter or said variable is greater than a second threshold, revert to instruction (a), the second threshold being larger than the first threshold.

6. A breathing assistance apparatus as claimed in claim 5, wherein if said change in said parameter or said variable is indicative of a decrease in flow a relatively short time delay is caused before said control means reverts to said instruction (b) and if said change indicates an increase inflow a relatively long time delay is caused before said control means reverts to said instruction (b).

7. A breathing assistance apparatus as claimed in claim 6 wherein said second threshold is based on the rate of change of said parameter or said variable with respect to time, and wherein when said rate of change goes over said second threshold said control means reverts to said instruction (a).

8. A breathing assistance apparatus as claimed in any preceding claim further comprising a gases supply means adapted to supply gases to said humidification means at a required pressure and resulting flow rate.

9. A breathing assistance apparatus as claimed in claim 8 wherein said gases supply means comprises a fan (150) driven by a variable speed electric motor.

**Patentansprüche**

1. Vorrichtung zur Unterstützung der Atmung, die zur Bereitstellung befeuchteter Gase mit einem gewünschten Feuchtigkeitsgehalt oder mit einer gewünschten Temperatur geeignet ist, welche Folgendes aufweist:

   ein Befeuchtungsmittel (1), welches eine Befeuchtungskammer (3) aufweist, die zur Aufnahme eines Wasservolumens geeignet ist, sowie ein Wassererhitzungsmittel (20) zum Erhitzen des Wassers zum Erzeugen von Wasserdampf innerhalb der verwendeten Kammer, wobei die Gase durch den Wasserdampf in der Kammer geleitet werden, wodurch sie befeuchtet werden, wobei das Befeuchtungsmittel eine elektrische Eingangsleistung aufweist und in der Lage ist, die Gase bis auf einen bestimmten Feuchtigkeitsgehalt zu befeuchten, wobei der Feuchtigkeitsgehalt von der Eingangsleistung zu dem Befeuchtungsmittel abhängt, und
   ein Kammerabtastmittel (8), welches eine Anzeige der Temperatur des Wassererhitzungsmittels (20) bereitstellt und eine Anzeige der elektrischen Leistung, die durch das Wassererhitzungsmittel gezogen wird, bereitstellt, **gekennzeichnet durch** ein Steuermittel (100), welches gespeicherte Anweisungen aufweist, um:

   (a) einen Parameter in Bezug auf die Flussrate der Gase **durch** die Vorrichtung zu bestimmen, wobei die Anweisung (a) Folgendes aufweist:

   i) Speisen des Wassererhitzungsmittels zum Erhitzen des Wassers in Richtung einer ersten Bedingung,
   ii) kontinuierliches Überwachen einer Variablen, die eine Eigenschaft des Wassererhitzungsmittels anzeigt, bis die Variable anzeigt, dass das Wasser im Wesentlichen die erste Bedingung erreicht hat, wobei die Variable die Anzeige der Temperatur des Wassererhitzungsmittels oder die Anzeige der **durch** das Wassererhitzungsmittel gezogenen Leistung anzeigt, und
   iii) Bestimmen des Parameters basierend mindestens auf der Variablen,

   (b) basierend auf mindestens dem Parameter, die erforderliche elektrische Eingangsleistung zu dem Befeuchtungsmittel zur Bereitstellung der Gase mit einem Feuchtigkeitsgehalt oder mit einer Temperatur im Wesentlichen ähnlich dem gewünschten Feuchtigkeitsgehalt oder der gewünschten Temperatur zu bestimmen; und
   (c) als die Eingangsleistung zu dem Befeuchtungsmittel ein Leistungsniveau zu liefern, das im Wesentlichen ähnlich der bestimmten Eingangsleistung zu dem Befeuchtungsmittel ist.

2. Vorrichtung zur Unterstützung der Atmung nach Anspruch 1, welche ferner ein Transportpfadmittel (6) zum Befördern der befeuchteten Gase von dem Befeuchtungsmittel zu einem Patienten aufweist.

3. Vorrichtung zur Unterstützung der Atmung nach Anspruch 2, welche ferner Folgendes aufweist:

   ein Pfaderhitzungsmittel (10), welches eine elektrische Eingangsleistung aufweist und mit dem Transportpfadmittel (6) in Zusammenhang steht, wobei Gase, die durch das Transportpfadmittel strömen, entweder direkt oder indirekt, durch das Pfaderhitzungsmittel erhitzt werden, wobei das Erhitzungsniveau von der Eingangsleistung zu dem Pfaderhitzungsmittel abhängt; und
   einen Umgebungstemperatursensor, der eine Anzeige der Außentemperatur bereitstellt;
   wobei die Anweisung (b) ferner eine Anweisung aufweist, um basierend auf mindestens der Anzeige der Außentemperatur die erforderliche Eingangsleistung zu dem Pfaderhitzungsmittel zu bestimmen, zur Bereitstellung der Gase an den Patienten mit einem Feuchtigkeitsgehalt oder mit einer Temperatur, der/die im Wesentlichen ähnlich dem gewünschten Feuchtigkeitsgehalt oder der gewünschten Temperatur ist;
   und wobei die Anweisung (c) ferner eine Anweisung aufweist, ein Leistungsniveau als die Eingangsleistung zu dem Pfaderhitzungsmittel bereitzustellen, das im Wesentlichen ähnlich der bestimmten Eingangsleistung zu dem Pfaderhitzungsmittel ist.

4. Vorrichtung zur Unterstützung der Atmung nach Anspruch 3, wobei die Bestimmung der Leistung zu dem Befeuchtungsmittel in Anweisung (b) auf der Anzeige der Außentemperatur basiert.

5. Vorrichtung zur Unterstützung der Atmung nach Anspruch 3, wobei das Steuermittel eine weitere Anweisung speichert, um:

   (d) den Parameter oder die Variable kontinuierlich zu überwachen, und, wenn eine Veränderung des Parameters oder der Variablen größer als ein erster Schwellenwert ist, zu Anweisung (b) zurückzukehren, und, wenn eine Veränderung des Parameters oder der Variablen größer als ein zweiter Schwellenwert ist, zu Anweisung (a) zurückzukehren, wobei der zweite Schwellenwert höher ist als der erste Schwellenwert.

6. Vorrichtung zur Unterstützung der Atmung nach Anspruch 5, wobei, wenn die Veränderung des Parameters oder der Variablen eine Abnahme im Fluss anzeigt, eine relativ kurze Zeitverzögerung veranlasst wird, bevor das Steuermittel zu Anweisung (b) zurückkehrt, und, wenn die Veränderung einen Anstieg im Fluss anzeigt, eine relativ lange Zeitverzögerung veranlasst wird, bevor das Steuermittel zu Anweisung (b) zurückkehrt.

7. Vorrichtung zur Unterstützung der Atmung nach Anspruch 6, wobei der zweite Schwellenwert auf der Veränderungsgeschwindigkeit des Parameters oder der Variablen in Bezug auf die Zeit basiert, und wobei, wenn die Veränderungsgeschwindigkeit den zweiten Schwellenwert übersteigt, das Steuermittel zu Anweisung (a) zurückkehrt.

8. Vorrichtung zur Unterstützung der Atmung nach einem der vorhergehenden Ansprüche, welche ferner ein Gasbereitstellungsmittel aufweist, das zur Bereitstellung von Gasen mit einem erforderlichen Druck und der daraus resultierenden Flussrate an das Befeuchtungsmittel geeignet ist.

9. Vorrichtung zur Unterstützung der Atmung nach Anspruch 8, wobei das Gasbereitstellungsmittel einen Lüfter (150) angetrieben durch einen drehzahlgeregelten Elektromotor aufweist.

## Revendications

1. Appareil d'assistance respiratoire conçu pour délivrer des gaz humidifiés à un niveau souhaité d'humidité ou à une température souhaitée, l'appareil comprenant :

   des moyens d'humidification (1) comprenant une chambre d'humidification (3) conçue pour recevoir un volume d'eau et des moyens (20) de chauffage d'eau qui chauffent ladite eau pour produire en utilisation de la vapeur d'eau dans ladite chambre, lesdits gaz qui traversent ladite vapeur d'eau présente dans ladite chambre étant de cette façon humidifiés,
   lesdits moyens d'humidification recevant de l'énergie électrique et étant capables d'humidifier lesdits gaz jusqu'à un niveau d'humidité qui dépend de ladite énergie introduite dans lesdits moyens d'humidification et
   des moyens (8) de détection de la chambre fournissant une indication de la température desdits moyens (20) de chauffage de l'eau et fournissant une indication de l'énergie électrique prélevée par lesdits moyens de chauffage de l'eau
   **caractérisé par**
   des moyens de contrôle (100) dans lesquels sont enregistrées des instructions permettant de :

      (a) déterminer un paramètre lié au débit desdits gaz dans ledit appareil, lesdites instructions (a) comprenant les étapes qui consistent à :

         i) alimenter lesdits moyens de chauffage pour chauffer ladite eau jusqu'à un premier état,
         ii) surveiller en continu une variable indicatrice d'une propriété desdits moyens de chauffage de l'eau jusqu'à ce que ladite variable indique que ladite eau a essentiellement atteint le premier état, ladite variable étant indicatrice de ladite indication de la température desdits moyens de chauffage ou de ladite indication de l'énergie prélevée par lesdits moyens de chauffage de l'eau et
         iii) déterminer ledit paramètre au moins sur la base de ladite variable,

      (b) déterminer au moins sur la base dudit paramètre la quantité d'énergie électrique requise pour que lesdits moyens d'humidification puissent délivrer lesdits gaz à un niveau d'humidité ou à une température essentiellement similaires audit niveau souhaité d'humidité ou à ladite température souhaitée et
      (c) délivrer auxdits moyens d'humidification ladite énergie à un niveau essentiellement similaire à ladite quantité d'énergie déterminée comme devant être délivrée auxdits moyens d'humidification.

2. Appareil d'assistance respiratoire selon la revendication 1, comprenant de plus des moyens (6) de parcours de transport qui transfèrent à un patient lesdits gaz humidifiés par lesdits moyens d'humidification.

3. Appareil d'assistance respiratoire selon la revendication 2, comprenant de plus :

des moyens (10) de chauffage de parcours aptes à recevoir de l'énergie électrique et associés auxdits moyens (6) de parcours de transport, les gaz s'écoulant dans lesdits moyens de parcours de transport étant chauffés directement ou indirectement par lesdits moyens de chauffage de parcours, le niveau de chauffage dépendant de ladite énergie délivrée auxdits moyens de chauffage de parcours, et

un capteur de température ambiante fournissant l'indication de la température extérieure,

ladite instruction (b) comprenant de plus une instruction permettant de déterminer sur la base d'au moins ladite indication de la température extérieure l'énergie qui doit être délivrée auxdits moyens de chauffage de parcours pour délivrer audit patient ledit gaz à un niveau d'humidité ou à une température essentiellement similaires audit niveau d'humidité ou à ladite température souhaités,

ladite instruction (c) comprenant de plus une instruction d'apport de ladite énergie auxdits moyens de chauffage de parcours à un niveau essentiellement similaire à ladite quantité d'énergie déterminée comme devant être délivrée auxdits moyens de chauffage de parcours.

4. Appareil d'assistance respiratoire selon la revendication 3, comprenant de plus la détermination de ladite énergie auxdits moyens d'humidification au cours de l'instruction (b) sur la base de ladite indication de la température extérieure.

5. Appareil d'assistance respiratoire selon la revendication 3, dans lequel lesdits moyens de contrôle enregistrent une instruction supplémentaire qui permet de :

(d) surveiller en continu ledit paramètre ou ladite variable et revenir à ladite instruction (b) lorsqu'un changement dudit paramètre ou de ladite variable est plus grand qu'une première valeur de seuil et revenir à l'instruction (a) lorsqu'un changement dudit paramètre ou de ladite variable est plus grand qu'une deuxième valeur de seuil, la deuxième valeur de seuil étant plus grande que la première valeur de seuil.

6. Appareil d'assistance respiratoire selon la revendication 5, dans lequel si ledit changement dudit paramètre ou de ladite variable est indicatif d'une diminution du débit, un retard de temps relativement court est induit avant que lesdits moyens de contrôle reviennent à ladite instruction (b) et si le changement est indicatif d'une augmentation du débit, un délai de durée relativement longue est induit avant que lesdits moyens de contrôle reviennent à ladite instruction (b).

7. Appareil d'assistance respiratoire selon la revendication 6, dans lequel la deuxième valeur de seuil est basée sur le taux de changement dudit paramètre ou de ladite variable en fonction du temps et dans lequel lesdits moyens de contrôle reviennent à ladite instruction (a) lorsque ledit taux de changement est supérieur à ladite deuxième valeur de seuil.

8. Appareil d'assistance respiratoire selon l'une quelconque des revendications précédentes, comprenant des moyens d'approvisionnement en gaz conçus pour effectuer l'approvisionnement en gaz dudit moyen d'humidification à une pression requise et au débit qui en résulte.

9. Appareil d'assistance respiratoire selon la revendication 8, dans lequel lesdits moyens d'approvisionnement en gaz comprennent une soufflante (150) entraînée par un moteur électrique à vitesse variable.

FIG 1

FIG 2

FIG 3

128— Energise
Heater plate 40°C
Conduit element 50%

130— Heater plate
stabilised?

no

yes

136— Calculate
gas flow

138— Calculate
heater plate
power

140— Calculate
conduit element
power

142— Has flow rate
changed

no

a little     a lot

144 —     146

FIG 4

FIG. 5

EP 1 207 929 B1

FIG. 6

EP 1 207 929 B1

FIG. 7

EP 1 207 929 B1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0885623 A2
- US 5640951 A, Fisher and Paykel

- US 56409511 B
- US 6050260 A
- US 5740795 A